# EUROPÄISCHE PATENTSCHRIFT

(11) **EP 2 046 702 B1**
(45) Veröffentlichungstag und Bekanntmachung des Hinweises auf die Patenterteilung: **09.12.2009**
(21) Anmeldenummer: 07802378.5
(22) Anmeldetag: 16.07.2007
(51) Int. Cl.: C07B 63/00, C07C 29/149, C07C 209/48, C07C 31/20

(54) **VERFAHREN ZUR AUFARBEITUNG VON LÖSUNGSMITTEL ENTHALTENDEN HYDRIERAUSTRÄGEN**
PROCESS FOR WORKING UP SOLVENT-CONTAINING HYDROGENATION PRODUCT MIXTURES
PROCÉDÉ DE RECONDITIONNEMENT DE PRODUITS D'HYDROGÉNATION CONTENANT UN SOLVANT

(30) Priorität: 25.07.2006 EP 06117797
(43) Veröffentlichungstag der Anmeldung: 15.04.2009
(73) Patentinhaber: BASF SE, 67056 Ludwigshafen (DE)
(72) Erfinder: PINKOS, Rolf, 67098 Bad Dürkheim (DE); HEIMANN, Jens, 67550 Worms (DE); POLKA, Hans-Martin, 69469 Weinheim (DE); URTEL, Heiko, 68165 Mannheim (DE); WINDECKER, Gunther, 67067 Ludwigshafen (DE)
(86) Internationale Anmeldenummer: PCT/EP2007/057325
(87) Internationale Veröffentlichungsnummer: WO 2008/012229

(56) Entgegenhaltungen:
- DE-A1- 10 341 612
- US-A- 4 254 059
- US-B1- 6 265 596
- DATABASE WPI Week 199302 Derwent Publications Ltd., London, GB; AN 1993-012154 XP002461518 & JP 04 338346 A (KAWASAKI STEEL CORP) 25. November 1992 (1992-11-25)

## Beschreibung

Die vorliegende Erfindung betrifft ein Verfahren zum Abtrennen von Lösungsmitteln aus lösungsmittelhaltigen Hydrierausträgen aus katalytischen Hydrierungen mit wasserstoffhaltigen Gasen, die mindestens ein eine Alkohol- Lacton-, Ether-, Lactam- oder Aminogruppe aufweisendes, wasserlösliches Hydrierprodukt in einem mit Wasser nicht oder nicht vollständig mischbaren Lösungsmittel mit einem Siedepunkt unter 100°C enthalten, wobei dem Hydrieraustrag Wasser zugegeben, die Lösungsmittel enthaltende Phase abgetrennt und in die Hydrierung zurückführt wird.

Die katalytische Hydrierung mit Wasserstoff ist ein weit verbreitetes Verfahren in der Chemie. Werden Verbindungen hydriert, die wasserlösliche Hydrierprodukte ergeben, so wird überwiegend ohne Lösungsmittel gearbeitet. Beispiele für in der industriellen organischen Chemie wichtige Verbindungen, deren Hydrierungen in der in Gas- oder Flüssigphase zu wasserlöslichen Verbindungen durchgeführt werden können, sind zum Beispiel Kohlenmonoxid, Ester, Säuren, Lactone, Anhydride, Aldehyde, Ketone, Nitrile, Amide und Aminosäuren. Da die Reaktionsgeschwindigkeiten unter diesen Bedingungen klein sind, werden Raum-Zeit-Ausbeuten unter 1 kg Produkt/Liter Reaktionsraum x h erzielt.

Es sind jedoch, beispielsweise aus US 6,265,596, Hydrierverfahren für wasserlösliche Verbindungen bekannt, bei denen die Reaktionsgeschwindigkeit und damit die Raum-Zeit-Ausbeute stark erhöht werden konnte, so dass sich der bei großtechnischen Anwendungen teure Reaktionsraum und die benötigte Katalysatormenge deutlich verringern lassen.

In diesen Verfahren wird in Gegenwart von Lösungsmitteln mit niedrigem Siedepunkt unter Druck hydriert und zur Gewinnung des Produkts das Reaktionsgemisch entspannt, zumeist auf Normaldruck. Die Rückführung des Lösungsmittels, v.a. wenn bei hohen Drücken hydriert wird, ist problematisch, so dass der Vorteil des geringen Reaktionsraums und der geringen notwendigen Katalysatormenge wieder zunichte gemacht werden kann.

Nach der Hydrierung wird der Hydrieraustrag, der überschüssigen Wasserstoff, Produkt, gegebenenfalls gebildete Zwischen- und Nebenprodukte, nicht umgesetztes Edukt und gegebenenfalls das Lösungsmittel, wenn in Gegenwart eines solchen hydriert wurde, enthält, in der Regel vor weiteren Aufarbeitungsschritten abgekühlt und entspannt. Dabei fallen Wasserstoff und gegebenenfalls Lösungsmittel gasförmig an. Sofern das Lösungsmittel unter den Verfahrensbedingungen der Wasserstoffabscheidung flüssig ist, wird es zusammen mit dem Produktgemisch, zumeist destillativ aufgetrennt. Das Lösungsmittel fällt dann als Leichtsieder, entweder flüssig oder gasförmig, an.

Das zurückgewonnene Lösungsmittel kann entweder verworfen oder, wie bei auf Wirtschaftlichkeit ausgerichteten großtechnischen Verfahren bevorzugt, zurückgeführt werden. Die Rückführung des Lösungsmittels erfordert zunächst jedoch die Aufwendung von erheblichen Mengen Kompressionsenergie.

Da die Hydrierung bei hohen Drücken erfolgt, muss das Lösungsmittel von einem niedrigen Druckniveau wieder auf den Reaktionsdruck komprimiert werden. Insbesondere bei Lösungsmitteln, die bei Umgebungstemperatur normalerweise gasförmig sind, ist dies energieaufwändig und deshalb wirtschaftlich nachteilig. Darüber ist es erforderlich, die Rückführkomponenten Lösungsmittel und Wasserstoff wieder auf das für die Hydrierung erforderliche Temperatur-Ausgangsniveau anzuheben, was wiederum energieaufwändig ist.

Der vorliegenden Erfindung liegt daher die Aufgabe zugrunde, ein Verfahren bereitzustellen, das ein effektives Abtrennen des Lösungsmittels aus durch katalytische Hydrierung erhaltenen Lösungsmittel enthaltenden Hydrierausträgen, die wasserlösliche Hydrierprodukte erhalten, ermöglicht. Die Trennung sollte so effektiv sein, dass die Rückführung des Lösungsmittels in die Hydrierung wirtschaftlich ist.

Es wurde nun überraschend ein Verfahren zur Abtrennung von Lösungsmitteln aus lösungsmittelhaltigen Hydrierausträgen aus katalytischen Hydrierungen mit wasserstoffhaltigen Gasen, die mindestens ein eine Alkohol- Lacton-, Ether-, Lactam- oder Aminogruppe aufweisendes, wasserlösliches Hydrierprodukt in einem mit Wasser nicht oder nicht vollständig mischbaren Lösungsmittel mit einem Siedepunkt unter 100°C enthalten, gefunden, das dadurch gekennzeichnet ist, dass dem Hydrieraustrag Wasser zugegeben, die Lösungsmittel enthaltende Phase abgetrennt und in die Hydrierung zurückführt wird.

Unter Hydrieraustrag wird das dem Hydrierreaktor entnommene Reaktionsgemisch nach der Hydrierung ohne weitere Aufrbeitungsschritte wie Destillationen verstanden. Der Hydrieraustrag enthält. Cas Lösungsmittel und das Hydrierprodukt sowie gegebenenfalls neben überschüssigem Wasserstoff, gegebenenfalls gebildete Zwischen- und Nebenprodukte und nicht umgesetztes Edukt. In der vorliegenden Anmeldung wird unter Hydrierprodukt das durch die Hydrierung herzustellende wasserlösliche Zielprodukt oder Gemische mehrere Zielprodukte verstanden. Wasserlösliche Hydrierprodukte im Sinne dieser Anmeldung sind beispielweise Methanol, Ethanol, Propanol, Butanol, iso-Butanol, Ethylenglycol, 1.2-Propylenglycol und 1.3-Butandiol, 1,4-Butandiol, 1,2,4-Butantriol, Tetrahydrofuran, 2-Methyltetrahydrofuran, 3-Methyltetrahydrofuran, gegebenenfalls methylsubstituiertes gamma-Butyrolacton, 1,5-Pentandiol, 1,6-Hexandiol, 1.4-Pentandiol, 2,5-(Bishydroxymethyl)tetrahydrofuran ,1,12-Dodecandiol, Pyrrolidon, N-Alkylpyrrolidone, C-alkylierte Pyrrolidone, C- und N-alkylierte Pyrrolidone, N-alkylierte Mono- und Diamine sowie Aminoalkohole oder deren Gemisch. Bevorzugte wasserlösliche Hydrierprodukte sind 1,4-Butandiol, Tetrahydrofuran, gamma-Butyrolacton, 1,5-Pentandiol, 1,6-Hexandiol, 1,4-Pentandiol, 2,5(Bishydroxymethyl)-tetrahydrofuran, Pyrrolidon, N-Alkylpyrrolidone, C-alkylierte Pyrrolidone, C- und N-alkylierte Pyrrolidone sowie Aminoalkohole oder deren Gemisch.

Die Produkte, die nach dem erfindungsgemäßen Verfahren entstehen, sind z.B. gesuchte Lösungsmittel, wie z.B. Tetrahydrofuran oder Ausgangsstoffe für Polyester wie 1,4-Butandiol oder 1,6-Hexandiol.

Erfindungsgemäß verwendbar sind Lösungsmittel, die mit Wasser unter den Druck- und Temperaturbedingungen des erfindungsgemäßen Verfahrens nicht oder nicht vollständig mischbar sind und die einen Siedepunkt bei einem Druck von 1 bar unter 100°C, bevorzugt unter 70°C, besonders bevorzugt unter 50°C, ganz besonders bevorzugt unter 25°C aufweisen.

Das Gewichtsverhältnis von Lösungsmittel zu Hydrierprodukt im Hydrieraustrag liegt zwischen 0,1 und 1000 , bevorzugt zwischen 0.5 und 100, besonders bevorzugt zwischen 1 und 50.

Beispiele für erfindungsgemäß verwendbare Lösungsmittel sind Kohlendioxid, Argon, Stickstoff, Kohlenwasserstoffe wie Methan, Ethan, Propan, Butan, iso-Butan, Pentan und seinen Isomere, Cyclopentan, Hexan und seinen Isomere, Cyclohexan, Heptan und seinen Isomeren, Aromaten wie Benzol, Toluol, Ether wie Dimethylether, Methylethylether, Diethylether, Dipropylether, Methylproplylether, Methyl-iso-propylether, Ethylpropylether, Ethyl-iso-propylether, Dibutylether, Methyl-tert.-butylether, Methyl-n-oder iso-butylether, Ethyl-n oder iso-butylether, Propyl-n oder iso-Butylether, iso- Propyl-n oder iso-Butylether. Die vorgenannten Lösungsmittel können ganz oder teilweise fluoriert sein. Bevorzugt sind Kohlenwasserstoffe mit 1 bis 6 Kohlenstoffatomen, Dialkylether mit bis zu 8 Kohlenstoffen, besonders bevorzugt sind Propan, Butan, Pentan, Dimethylether. Diethylether und Methyl-tert.-bulylether sowie Kohlendioxid als Lösungsmittel für cas enfindungsgemäße Verfahren.

Dem Hydrieraustrag wird eine die Phasentrennung bewirkende Menge an Wasser oder, falls sich bereits eine schwach ausgeprägte Phasentrennung einstellt, eine die Phasentrennung vervollständigende Menge an Wasser zugegeben. Diese Menge an Wasser beträgt 0,01 bis 1000 Gew.-% Wasser, bevorzugt 50 bis 300 Gew.-% Wasser, besonders bevorzugt 100 bis 200 Gew.-% Wasser, jeweils bezogen auf den Hydrieraustrag, zugegeben.

Während der erfindungsgemäßen Phasenscheidung liegt der Druck bei einem dem Druck der vorangegangenen Hydrierung entsprechenden Wert oder bis zu 50 bar niedriger. Der Druck der Phasenscheidung liegt bevorzugt bei 10 bis 400 bar, bevorzugt bei 50 bis 350 bar und besonders bevorzugt bei 75 und 300 bar. In einer besonders bevorzugten Ausführungsform des Verfahrens liegt der Druck bei der Phasentrennung auf oder bis zu 20 bar unterhalb des Druckes der vorgeschalteten Hydrierung.

Die Phasentrennung kann bei der am Hydrierreaktorausgang herrschenden Temperatur der dem erfindungsgemäßen Verfahren vorangegangenen Hydrierung oder bei tieferer Temperatur, bevorzugt bei 5 bis 250°C tiefer durchgeführt werden. Ganz bevorzugt wird derHydrieraustrag zur Phasentrennung auf die Temperatur abgekühlt, die am Ausgang des Hydrierreaktors herrscht. Die erfindungsgemäße Phasentrennung wird in an sich für diesen Zweck bekannten Apparaturen wie z. B. Phasenscheidern oder Dekantern durchgeführt.

Während die Wasserphase vorwiegend das Hydrierprodukt enthält, weist die das Löungsmittel enthaltende Phase (Lösungsmittelphase) neben dem Lösungsmittel und Wasserstoff gegebenfalls nicht umgesetztes Hydrieredukt, Zwischenprodukte der Hydrierung, geringe Mengen des Hydrierproduktes und Nebenprodukte der Hydrierung auf.

Die Aufarbeitung der das Hydrierprodukt enthaltenden Wasserphase erfolgt nach an sich bekannten Verfahren wie beispielsweise durch Kristallisation und/oder Destillation. In der Regel wird zumindest das Wasser destillativ vom Hydrierprodukt abgetrennt. Vorteilhafterweise werden dabei die Destillationsbedingungen so wählt, dass aufgrund der für die Phasenscheidung gewählten Druck- und Temperaturbedingungen, entweder Wasser oder das Hydrierprodukt beim Eintritt in die Destillationskolonne verdampft. Das abgetrennte Wasser wird bevorzugt in die Phasentrennung zurückgeführt.

Die erfindungsgemäße Phasentrennung kann kontinuierlich oder diskontinuierlich zusammen mit einer vorgeschalteten Hydrierung betrieben werden, wobei die kontinuierliche Fahrweise bevorzugt ist.

In einer bevorzugten Ausführungsform wird das abgetrennte Lösungsmittel ohne Aufarbeitung wieder in die Hydrierung zurückgefahren. Dabei ist es bevorzugt, das Edukt der Hydrierung, bevor es den Katalysator erreicht, vollständig oder zumindest nahezu vollständig mit diesem Lösungsmittel zu vermischen.

Als wasserstoffhaltige Gase sind neben Wasserstoff auch dessen Gemische mit weiteren unter Reaktionsbedingungen inerten Gasen, wie zum Beispiel Stickstoff, für das erfindungsgemäße Verfahren geeignet. Bevorzugt wird Wasserstoff verwendet.

Die Hydrierung erfolgt bevorzugt an heterogenen Katalysatoren, die besonders bevorzugt im Reaktor fest angeordnet sind. Als Katalysatoren können in dem Hydrierverfahren solche eingesetzt werden, die mindestens eines der folgenden Elemente als Metall und/oder als Verbindung wie zum Beispiel als Oxid enthalten: Fe, Cu, Ag, Au, Ni, Pd, Pt, Co, Rh, Ir, Ru, Mn, Re, Cr oder Mo. Bevorzugt enthält der Katalysator Fe, Cu, Co, Re, Ni, Ru, Pt oder Pd.

Geeignet ist insbesondere mindestens ein heterogener Katalysator, wobei mindestens eines der obenstehend genannten Metalle (Aktivmetalle) als Metall als solches, als Raney-Katalysator und/oder aufgebracht auf einen üblichen Träger eingesetzt werden kann. Werden zwei oder mehr Aktivmetalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salzen oder Carbonaten ausfällt, die erhaltenen Niederschläge anschließend trocknet und diese dann durch Calcinierung bei im allgemeinen 300 bis 700°C, insbesondere 400 bis 600°C in die entsprechenden Oxide, Mischoxide und/oder gemischt-valentigen Oxide umwandelt, die durch eine Behandlung mit Wasserstoff oder mit Wasserstoff enthaltenden Gasen im Bereich von im Allgemeinen 50 bis 700°C, insbesondere 100 bis 400°C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Füllung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytisch-hydrierend wirkende Komponente beispielsweise durch Imprägnierung auf ein Trägermaterial aufgebracht worden ist.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600°C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,01 bis 90 Gew.-%, vorzugsweise im Bereich von 0,1 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-A 25 19 817, EP-A 1 477 219 A1 oder EP-A 0 285 420 A1 auf den Träger aufgebracht werden. In den Katalysatoren gemäß den vorgenannten Schriften liegen die katalytisch aktiven Metalle als Legierungen vor, die durch thermische Behandlung und/oder Reduktion der z.B. durch Tränkung des Trägermaterials mit einem Salz oder Komplex der zuvor genannten Metalle, erzeugt werden.

Aufgrund der Toxizität chromhaltiger Katalysatoren werden bevorzugt chromfreie Katalysatoren eingesetzt. Selbstverständlich eignen sich technisch auch entsprechende, dem Fachmann bekannte chromhaltige Katalysatoren für einen Einsatz in dem erfindungsgemäßen Verfahren, wodurch sich jedoch nicht die gewünschten Vorteile, die insbesondere umwelt- und arbeitstechnischer Natur sind, ergeben.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien sowohl für Fällungskatalysatoren als auch für Trägerkatalysatoren können können die Oxide des Aluminiums und Titans, Zirkoniumdioxid, Siliciumdioxid, Tonerden wie beispielsweise Montmorillonite, Bentonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbare Katalysatoren dienen. Geeignet sind auch metallische Träger, auf denen das hydrieraktive Metall abgeschieden wurde, beispielsweise Cu auf dem z.B. Pd, Pt oder Ru aus den entsprechenden in Wasser gelösten Metallsalzen abgeschieden wurde.

Insbesondere bevorzugte erfindungsgemäße Katalysatoren sind Trägerkatalysatoren, die Fe, Cu, Co, Re; Ni, Pt und/oder Pd enthalten, wobei insbesondere bevorzugte Träger Aktivkohle, Aluminiumoxid, Titandioxid und/oder Siliziumdioxid oder deren Gemische sind.

Ein erfindungsgemäß einsetzbarer heterogener Katalysator wird bevorzugt als Festbettkatalysator in der erfindungsgemäßen Hydrierstufe eingesetzt werden, dabei werden die Katalysatoren bevorzugt als stückiges Material eingesetzt, d.h. als Formkörper beispielsweise in Form von Splitt, Ringen, Tabletten oder Extrudaten. Die Durchmesser der einzelnen Katalysatorformkörper liegen zwischen 0,01 und 15 mm, bevorzugt sind 0.02 und 10 mm, besonders bevorzugt sind 0,05 bis 5 mm.

Die erfindungsgemäße Hydrierung kann diskontinuierlich oder kontinuierlich z.B. in einem oder mehreren mit einer Katalysatorschüttung gefüllten Reaktorrohren durchgeführt werden, wobei die kontinuierliche Fahrweise bevorzugt ist. Besonders bevorzugt wird die erfindungsgemäße Hydrierung zusammen mit der erfindungsgemäßen Phasentrennung als Gesamtprozess kontinuierlich betrieben.

Die Hydrierung erfolgt auf einem Druck und Temperaturniveau, das mindestens dem der Phasenscheidung entspricht. Der Bezugspunkt für Druck und Temperatur ist dabei der Reaktorausgang. Beispielsweise liegen die Hydrierdrücke bei 10 bis 400 bar, bevorzugt bei 50 bis 350 bar und besonders bevorzugt bei 75 bis 300 bar. Die Temperatur der Hydrierung liegt bei 20 bis 370°C, bevorzugt bei 40 bis 350°C und besonders bevorzugt bei 70 bis 330°C.

Das erfindungsgemäße Verfahren wird anhand der nachstehenden Beispiel näher erläutert.

### Beispiele

### Beispiel 1:

Ein ölbeheizter Doppelmantelrohrreaktor (3 cm Innendurchmesser) wurde mit 3 ml eines Cu - Katalysators (60 % CuO, 30 % Al2O3, 10 % Mn2O3) in Form von 0,1 mm großen Formkörper befüllt. Der Katalysator wurde zunächst mit Stickstoff- / Wasserstoffgemischen, später mit reinem Wasserstoff bei 250°C und 250 bar aktiviert. Bei 250°C und 250 bar wurden über eine temperierte Rohrwendel 20 g/h Adipinsäuredimethylester, 180 g/h Dimethylether und 10 Mol Wasserstoff pro Mol Edukt über den Katalysator gepumpt.

Der Hydrieraustrag wies eine Temperatur von 270°C auf und gelangte über eine gekühlte Mischstrecke, vor der 100 g/h Wasser (25°C) eindosiert wurden, in einen Phasenscheider von 2 L Volumen. Die Temperatur im Phasenscheider betrug 50°C und der Druck 250 bar. Bei einem Füllstand des Phasenscheiders von ca. 50 % wurde die obere Phase, welche zu etwa 90 % aus Dimethylether bestand und den überwiegenden Teil des überschüssigen Wasserstoffs enthielt, kontinuierlich in den Hydrierreaktor zurückgepumpt und gleichzeitig der Zulauf von frischem Dimethylether auf 60 g/h und die Menge frischen Wasserstoffs auf 5 Mol / Mol Edukt reduziert. Die untere Phase aus dem Phasenscheider wurde über ein Druckhalteventil kontinuierlich abgelassen und nach dem Stand der Technik destillativ aufgetrennt. Zurückgewonnenes Wasser wurde wieder in die Phasenscheidung zurückgeführt.

Als Hydrierprodukt wurde in 96 %iger Ausbeute 1,6-Hexandiol gewonnen. Ferner wurden 2 % dimeres 1,6-Hexandiol sowie einige weitere mengenmäßig unbedeutende Nebenprodukte gefunden.

### Vergleichsbeispiel 1:

Beispiel 1 wurde wiederholt, allerdings wurde kein Wasser nach der Hydrierung zugesetzt, die Temperatur im Phasenscheider wurde durch Kühlung auf 50°C abgekühlt. Es konnten im Phasenscheider keine zwei flüssige Phasen beobachtet werden, d.h. das Lösungsmittel Dimethylether konnte nicht auf einfache Weise abgetrennt und zurückgeführt werden.

### Beispiel 2:

Ein ölbeheizter Doppelmantelrohrreaktor (3 cm Innendurchmesser) wurde mit 10 ml eines Co / Mn - Katalysators (32 % CoO, 58 % Co, 10 % Mn2O3) in Form von 0,1 mm großen Formkörper befüllt. Der Katalysator wurde zunächst mit Stickstoff- / Wasserstoffgemischen, später mit reinem Wasserstoff bei 280 °C und 250 bar aktiviert. Bei 150°C und 250 bar wurden über eine temperierte Rohrwendel 5 g/h Adipinsäuredinitril, 95 g/h n-Butan und 15 Mol Wasserstoff pro Mol Edukt über den Katalysator gepumpt. Der Hydrieraustrag wies eine Temperatur von 160°C auf und gelangte über eine gekühlte Mischstrecke, vor der 50 g/h Wasser (25°C) eindosiert wurden, in einen Phasenscheider von 2 L Volumen. Die Temperatur im Phasenscheider betrug 50°C und der Druck 250 bar. Bei einem Füllstand des Phasenscheiders von ca. 50 % wurde die obere Phase, welche zu über 95 % aus n-Butan bestand und den überwiegenden Teil des überschüssigen Wasserstoffs enthielt, kontinuierlich in den Hydrierreaktor zurückgepumpt und gleichzeitig der Zulauf von frischem n-Butan auf 1 g/h und die Menge an frischem Wasserstoff auf 5 Mol / Mol Edukt reduziert. Die untere Phase aus dem Phasenscheider wurde über ein Druckhalteventil kontinuierlich abgelassen. Es gasten nur sehr geringe Mengen Wasserstoff und n-Butan aus. Das restliche Produkt wurde nach dem Stand der Technik destillativ aufgereinigt. Zurückgewonnenes Wasser wurde wieder in die Phasentrennung zurückgeführt.
Als Hydrierprodukt wurde in 85 %iger Ausbeute 1,6-Diaminohexan gewonnen. Ferner wurden 5 % dimeres 1,6-Diaminohexan, 3 % 1-Azacycloheptan sowie einige weitere mengenmäßig unbedeutende Nebenprodukte gefunden.

### Vergleichsbeispiel 2:

Beispiel 2 wurde wiederholt, allerdings wurde kein Wasser nach der Hydrierung zugesetzt, die Temperatur im Phasenscheider wurde durch Kühlung ebenfalls auf ca. 50°C abgekühlt. Es konnten im Phasenscheider keine zwei flüssige Phasen beobachtet werden, d.h. das Lösungsmittel n-Butan konnte nicht auf einfache Weise abgetrennt und zurückgeführt werden.

### Beispiel 3:

Es wurde der im Beispiel 1 beschriebene Reaktor mit dem gleichen Katalysator verwendet. Hier wurden bei 220°C und 250 bar über eine temperierte Rohrwendel 9 g/h Maleinsäureanhydrid (Schmelze), 81 g/h n-Butan und 10 Mol Wasserstoff pro Mol Edukt über den Katalysator gepumpt.

Der Hydrieraustrag wies eine Temperatur von 240°C auf und gelangte über eine gekühlte Mischstrecke, vor der 100 g/h Wasser (25°C) eindosiert wurden, in einen Phasenscheider von 2 L Volumen. Die Temperatur im Phasenscheider betrug 50°C und der Druck 250 bar. Bei einem Füllstand des Phasenscheiders von ca. 50 % wurde die obere Phase, welche zu über 95 % aus n-Butan bestand und den überwiegenden Teil des überschüssigen Wasserstoffs enthielt, kontinuierlich in den Hydrierreaktor zurückgepumpt und gleichzeitig der Zulauf von frischem n-Butan auf 2 g/h und die Menge an frischem Wasserstoff auf 6 Mol / Mol Edukt reduziert.

Die untere Phase aus dem Phasenscheider wurde über ein Druckhalteventil kontinuierlich abgelassen. Es gasten nur sehr geringe Mengen Wasserstoff und n-Butan aus. Das restliche Produkt wurde nach dem Stand der Technik destillativ aufgereinigt. Zurückgewonnenes Wasser wurde wieder in die Phasentrennung zurückgeführt.
Als Hydrierprodukt wurde in 85 %iger Ausbeute 1,4-Butandiol gewonnen. Ferner wurden 8 % Tetrahydrofuran, 5 % gamma-Butyrolacton sowie einige weitere mengenmäßig unbedeutende Nebenprodukte gefunden.

### Vergleichsbeispiel 3:

Beispiel 3 wurde wiederholt, allerdings wurde kein Wasser nach der Hydrierung zugesetzt, die Temperatur im Phasenscheider wurde durch Kühlung ebenfalls auf ca. 50°C abgekühlt. Im Phasenscheider waren 2 flüssige Phasen zu erkennen, jedoch war die Löslichkeit von Butan in der Produktphase so groß, dass ein Großteil des Butans über die Produktphase verloren ging und man zur Aufrechterhaltung eines stabilen Gleichgewichts, trotz Rückführung der Butanphase, 50 g/h Frischbutan dosieren musste.

## Patentansprüche

1. Verfahren zum Abtrennen von Lösungsmitteln aus lösungsmittelhaltigen Hydrierausträgen aus einem katalytischem Hydrierverfahren, die ein mindestens eine Alkohol-, Lacton-, Ether-, Lactam- oder Aminogruppe aufweisendes, wasserlösliches Hydrierprodukt in einem mit Wasser nicht oder nicht vollständig mischbaren Lösungsmittel mit einem Siedepunkt unter 100°C enthalten, **dadurch gekennzeichnet, dass** dem Hydrieraustrag Wasser zugegeben, die Lösungsmittel enthaltende Phase abgetrennt und in die Hydrierung zurückführt wird.

2. Verfahren nach Anspruch 1, **dadurch gekennzeichnet, dass** das wasserlösliche Hydrierprodukt ausgewählt ist aus Methanol, Ethanol, Propanol, Butanol, iso-Butanol, Ethylenglycol, 1,2-Propylenglycol und 1,3-Butandiol, 1,4-Butandiol, 1,2,4-Butantriol, Tetrahydrofuran, 2-Methyltetrahydrofuran. 3-Methyltetrahydrofuran, gegebenenfalls methylsubstituiertes gamma-Butyrolacton, 1,5-Pentandiol, 1,6-Hexandiol. 1,4-Pentandiol, 2,5-(Bishydroxymethyl)tetrahydrofuran ,1,12-Dodecandiol, Pyrrolidon, N-Alkylpyrrolidonen, C-alkylierten Pyrrolidonen, C- und N-alkylierten Pyrrolidonen, N-alkylierten Mono- und Diaminen sowie Aminoalkoholen oder deren Gemisch.

3. Verfahren nach Anspruch 1 oder 2, **dadurch gekennzeichnet, dass** das wasserlösliche Hydrierprodukt ausgewählt ist aus 1,4-Butandiol, Tetrahydrofuran, gegebenenfalls methylsubstituiertes gamma-Butyrolacton, 1,5-Pentandiol. 1,6-Hexandiol, 1,4-Pentandiol, 2,5-(Bishydroxymethyl)tetrahydrofuran, Pyrrolidon, N-Alkylpyrrolidonen, C-alkylierten Pyrrolidonen, C- und N-alkylierte Pyrrolidonen sowie Aminoalkoholen oder deren Gemisch.

4. Verfahren nach einem der Ansprüche 1 bis 3, **dadurch gekennzeichnet, dass** nach der Hydrierung 0,01 bis 1000 Gew.-% Wasser, bezogen auf den Hydrieraustrag zugegeben werden.

5. Verfahren nach einem der Ansprüche 1 bis 4, **dadurch gekennzeichnet, dass** die Phasentrennung bei Temperaturen unterhalb der Hydrieraustragstemperatur durchgeführt wird.

6. Verfahren nach einem der Ansprüche 1 bis 5, **dadurch gekennzeichnet, dass** die Phasentrennung bei Temperaturen durchgeführt wird, die 5 bis 250°C unter der Hydrieraustragstemperatur durchgeführt wird.

7. Verfahren nach einem der Ansprüche 1 bis 4, **dadurch gekennzeichnet, dass** die Phasentrennung bei Temperaturen, die der Hydrieraustragstemperatur entspricht, durchführt wird.

8. Verfahren nach einem der Ansprüche 1 bis 7, **dadurch gekennzeichnet, dass** die Phasentrennung bei dem Druck durchgeführt wird, bei dem auch hydriert wird, oder bis zu 50 bar darunter.

9. Verfahren nach einem der Ansprüche 1 bis 8, **dadurch gekennzeichnet, dass** das Lösungsmittel ausgewählt ist aus Kohlendioxid, Argon, Stickstoff, Kohlenwasserstoffen wie Methan, Ethan, Propan, Butan, iso-Butan, Pentan und seinen Isomeren, Cyclopentan, Hexan und seinen Isomeren, Cyclohexan, Heptan und seinen Isomeren, Aromaten wie Benzol, Toluol, Ethern wie. Dimethylether, Methylethylether, Diethylether, Dipropylether, Methylproplylether, Methyl-iso-propylether, Ethylpropylether, Ethyl-iso-propylether, Dibutylether, Methyl-tert.-butylether, Methyl-n- oder iso-butylether, Ethyl-n oder iso-butylether, Propyl-n oder iso-Butylether, iso-Propyl-n oder iso-Butylether.

10. Verfahren nach einem der Ansprüche 1 bis 9, **dadurch gekennzeichnet, dass**, das Lösungsmittel ausgewählt ist aus Kohlendioxid, Argon, Stickstoff, Propan, Butan und seinen Isomeren, iso-Butan, Pentan und seinen Isomeren, Dimethylether und Diethylether.

11. Verfahren nach einem der Ansprüche 1 bis 10 **dadurch gekennzeichnet, dass,** das Gewichtsverhältnis von Lösungsmittel zum Hydrierprodukt zwischen 0,1 und 1000 liegt.

12. Verfahren nach einem der Ansprüche 1 bis 11, **dadurch gekennzeichnet, dass** das Verfahren zum Abtrennen von Lösungsmitteln zusammen mit der vorgeschalteten katalytischen Hydrierung kontinuierlich betrieben wird.

## Claims

1. A process for separating off solvents from solvent-comprising hydrogenation outputs from a catalytic hydrogenation process, which comprise a water-soluble hydrogenation product having at least one alcohol, lactone, ether, lactam or amino group in a solvent which is immiscible or not completely miscible with water and has a boiling point below 100°C, wherein water is added to the hydrogenation output, the solvent-comprising phase is separated off and recirculated to the hydrogenation.

2. The process according to claim 1, wherein the water-soluble hydrogenation product is selected from among methanol, ethanol, propanol, butanol, isobutanol, ethylene glycol, 1,2-propylene glycol and 1,3-butanediol, 1,4-butanediol, 1,2,4-butanetriol, tetrahydrofuran, 2-methyltetrahydrofuran, 3-methyltetrahydrofuran, optionally methyl-substituted gamma-butyrolactone, 1,5-pentanediol, 1,6-hexanediol, 1,4-pentanediol, 2,5-bis(hydroxymethyl)tetrahydrofuran, 1,12-dodecanediol, pyrrolidone, N-alkylpyrrolidones, C-alkylated pyrrolidones, C- and N-alkylated pyrrolidones, N-alkylated monoamines and diamines and amino alcohols and mixtures thereof.

3. The process according to claim 1 or 2, wherein the water-soluble hydrogenation product is selected from among 1,4-butanediol, tetrahydrofuran, optionally methyl-substituted gamma-butyrolactone, 1,5-pentanediol, 1,6-hexanediol, 1,4-pentanediol, 2,5-bis(hydroxymethyl)tetrahydrofuran, pyrrolidone, N-alkylpyrrolidones, C-alkylated pyrrolidones, C- and N-alkylated pyrrolidones and amino alcohols and mixtures thereof.

4. The process according to any of claims 1 to 3, wherein from 0.01 to 1000% by weight of water, based on the hydrogenation output, are added after the hydrogenation.

5. The process according to any of claims 1 to 4, wherein the phase separation is carried out at temperatures below the hydrogenation output temperature.

6. The process according to any of claims 1 to 5, wherein the phase separation is carried out at temperatures which are from 5 to 250°C below the hydrogenation output temperature.

7. The process according to any of claims 1 to 4, wherein the phase separation is carried out at temperatures which correspond to the hydrogenation output temperature.

8. The process according to any of claims 1 to 7, wherein the phase separation is carried out at the pressure at which the hydrogenation is also carried out or up to 50 bar below this.

9. The process according to any of claims 1 to 8, wherein the solvent is selected from among carbon dioxide, argon, nitrogen, hydrocarbons such as methane, ethane, propane, butane, isobutane, pentane and its isomers, cyclopentane, hexane and its isomers, cyclohexane, heptane and its isomers, aromatics such as benzene, toluene, ethers such as dimethyl ether, methyl ethyl ether, diethyl ether, dipropyl ether, methyl propyl ether, methyl isopropyl ether, ethyl propyl ether, ethyl isopropyl ether, dibutyl ether, methyl-tert-butyl ether, methyl n-butyl or isobutyl ether, ethyl n-butyl or isobutyl ether, propyl n-butyl or isobutyl ether, isopropyl n-butyl or isobutyl ether.

10. The process according to any of claims 1 to 9, wherein the solvent is selected from among carbon dioxide, argon, nitrogen, propane, butane and its isomers, isobutane, pentane and its isomers, dimethyl ether and diethyl ether.

11. The process according to any of claims 1 to 10, wherein the weight ratio of solvent to hydrogenation product is from 0.1 to 1000.

12. The process according to any of claims 1 to 11, wherein the process for separating off solvents is operated continuously together with the preceding catalytic hydrogenation.

## Revendications

1. Procédé pour la séparation de solvants à partir de décharges d'hydrogénation, contenant des solvants, provenant d'un procédé d'hydrogénation catalytique, qui contiennent au moins un produit d'hydrogénation hydrosoluble, comportant au moins un groupe alcool, lactone, éther, lactame ou amino, dans un solvant non ou non totalement miscible à l'eau, ayant un point d'ébullition inférieur à 100 °C, **caractérisé en ce qu'**on ajoute de l'eau à la décharge d'hydrogénation, on sépare la phase contenant le solvant et on la renvoie dans l'hydrogénation.

2. Procédé selon la revendication 1, **caractérisé en ce que** le produit d'hydrogénation hydrosoluble est choisi parmi le méthanol, l'éthanol, le propanol, le butanol, l'isobutanol, l'éthylèneglycol, le 1,2-propylèneglycol et le 1,3-butanediol, le 1,4-butanediol, le 1,2,4-butanediol, le tétrahydrofuranne, le 2-méthyltétrahydrofuranne, le 3-méthyltétrahydrofuranne, une gamma-butyrolactone éventuellement substituée par méthyle, le 1,5-pentanediol, le 1,6-hexanediol, le 1,4-pentanediol, le 2,5-(bishydroxyméthyl)tétrahydrofuranne, le 1,12-dodécanediol, la pyrrolidone, des N-alkylpyrrolidones, des pyrrolidones C-alkylées, des pyrrolidones C- et N-alkylées, des mono- et diamines N-alkylées ainsi que des aminoalcools ou un mélange de ceux-ci.

3. Procédé selon la revendication 1 ou 2, **caractérisé en ce que** le produit d'hydrogénation hydrosoluble est choisi parmi le 1,4-butanediol, le tétrahydrofuranne, une gamma-butyrolactone éventuellement substituée par méthyle, le 1,5-pentanediol, le 1,6-hexanediol, le 1,4-pentanediol, le 2,5-(bishydroxyméthyl)-tétrahydrofuranne, la pyrrolidone, des N-alkylpyrrolidones, des pyrrolidones C-alkylées, des pyrrolidones C- et N-alkylées ainsi que des aminoalcools ou un mélange de ceux-ci.

4. Procédé selon l'une quelconque des revendications 1 à 3, **caractérisé en ce qu'**après l'hydrogénation on ajoute de 0,01 à 1 000 % en poids d'eau, par rapport à la décharge d'hydrogénation.

5. Procédé selon l'une quelconque des revendications 1 à 4, **caractérisé en ce qu'**on effectue la séparation de phases à des températures inférieures à la température de la décharge d'hydrogénation.

6. Procédé selon l'une quelconque des revendications 1 à 5, **caractérisé en ce qu'**on effectue la séparation de phases à des températures qui sont inférieures de 5 à 250 °C à la température de la décharge d'hydrogénation.

7. Procédé selon l'une quelconque des revendications 1 à 4, **caractérisé en ce qu'**on effectue la séparation des phases à des températures qui correspondent à la température de la décharge d'hydrogénation.

8. Procédé selon l'une quelconque des revendications 1 à 7, **caractérisé en ce qu'**on effectue la séparation des phases sous la pression sous laquelle on effectue également l'hydrogénation ou sous une pression de jusqu'à 50 bars inférieure à celle-ci.

9. Procédé selon l'une quelconque des revendications 1 à 8, **caractérisé en ce que** le solvant est choisi parmi le dioxyde de carbone, l'argon, l'azote, des hydrocarbures tels que le méthane, l'éthane, le propane, le butane, l'isobutane, le pentane et ses isomères, le cyclopentane, l'hexane et ses isomères, le cyclohexane, l'heptane et ses isomères, des composés aromatiques tels que le benzène, le toluène, des éthers tels que l'éther diméthylique, l'oxyde de méthyle et d'éthyle, l'oxyde d'éthyle, l'éther dipropylique, l'oxyde de méthyle et de propyle, l'oxyde de méthyle et d'isopropyle, l'oxyde d'éthyle et de propyle, l'oxyde d'éthyle et d'isopropyle, l'éther dibutylique, l'oxyde de méthyle et de tert-butyle, l'oxyde de méthyle et de n- ou isobutyle, l'oxyde d'éthyle et de n- ou isobutyle, l'oxyde de propyle et de n- ou isobutyle, l'oxyde d'isopropyle et de n- ou isobutyle.

10. Procédé selon l'une quelconque des revendications 1 à 9, **caractérisé en ce que** le solvant est choisi parmi le dioxyde de carbone, l'argon, l'azote, le propane, le butane et ses isomères, l'isobutane, le pentane et ses isomères, l'éther diméthylique et l'oxyde d'éthyle.

11. Procédé selon l'une quelconque des revendications 1 à 10, **caractérisé en ce que** le rapport pondéral du solvant au produit d'hydrogénation est compris entre 0,1 et 1 000.

12. Procédé selon l'une quelconque des revendications 1 à 11, **caractérisé en ce** le procédé pour la séparation de solvants est effectué en continu conjointement avec l'hydrogénation catalytique effectuée en amont.
